# EUROPÄISCHE PATENTSCHRIFT

(11) **EP 1 753 514 B1**
(45) Veröffentlichungstag und Bekanntmachung des Hinweises auf die Patenterteilung: **02.05.2012**
(21) Anmeldenummer: 05741673.7
(22) Anmeldetag: 29.04.2005
(51) Int. Cl.: A61K 8/41, A61K 8/39, A61K 8/45, A61K 8/86

(54) **HAUTREINIGUNGSMITTEL, INSBESONDERE ZUR ENTFERNUNG VON DRUCKFARBEN UND/ODER TINTENVERSCHMUTZUNGEN**
SKIN CLEANSING AGENT, PARTICULARLY FOR REMOVING PRINTING INKS AND/OR SOILING CAUSED BY INK
AGENT NETTOYANT POUR LA PEAU, NOTAMMENT POUR ELIMINER DES ENCRES D'IMPRIMERIE ET/OU DES SALISSURES DUES A L'ENCRE

(30) Priorität: 28.05.2004 DE 102004026684
(43) Veröffentlichungstag der Anmeldung: 21.02.2007
(73) Patentinhaber: Evonik Stockhausen GmbH, 47805 Krefeld (DE)
(72) Erfinder: BLÄSER, Edeltraut, 47799 Krefeld (DE); VEEGER, Marcel, 47874 Goch (DE); ZUR MÜHLEN, Annette, 47803 Krefeld (DE); THOERNER, Brigitte, 40235 Düsseldorf (DE)
(86) Internationale Anmeldenummer: PCT/EP2005/004633
(87) Internationale Veröffentlichungsnummer: WO 2005/117826

(56) Entgegenhaltungen:
- WO-A-01/30315
- WO-A-99/06021
- WO-A-99/19432
- WO-A-03/026609
- WO-A-03/037270
- DE-A1- 19 918 188
- US-A- 5 104 567

## Beschreibung

Die Erfindung betrifft ein Hautreinigungsmittel, insbesondere zur Reinigung von extremen Haut- und Handreinigungsverschmutzungen, die beispielsweise durch reduzierbare oder oxidierbare Druckfarben und/oder Tinten, insbesondere Druckertinten hervorgerufen werden.

Haut- und Handreinigungsmittel werden in der Industrie umfangreich eingesetzt, insbesondere dort, wo hartnäckige Verschmutzungen auftreten, die durch Lacke, Fette, Öle, Schmierstoffe, Metallstäube, Graphit, Ruß aber auch Druckfarben und/oder Tinten hervorgerufen werden.

Derartige Reinigungsmittel sind beispielsweise als sogenannte Grobhandreiniger bekannt (vgl. H. Tronnier, J. Kresken, K. Jablonski, B. Komp, "Haut und Beruf", Grosse Verlag, Berlin, S.75 - 108 [1989]). Üblicherweise handelt es sich hierbei um Zubereitungen, die ein Abrasivum, Tensid/Tensidgemische, Verdickungsmittel sowie gegebenenfalls Hilfsstoffe zur Regulierung der Konsistenz, von Aussehen, Geruch und Stabilität, wie Pigmente, Duftstoffe, Stabilisatoren und Konservierungsmittel, enthalten. Für besonders hartnäckige Verschmutzungen gibt es Produkte, bei denen die Verwendung der oben genannten Inhaltsstoffe nicht ausreicht. Diesen Zubereitungen werden organische Lösungsmittel zugesetzt, wie z.B. aliphatische Kohlenwasserstoffe, Terpene, Carbonsäureester vom Typ Dimethyladipat, Dimethylglutarat, Dimethylsuccinat (DBE) und Di-n-butyladipat bzw. Di-isopropyladipat, wie sie in der DE 43 35933 A 1 beschrieben worden sind.

Weiterhin ist auf die im Markt erhältlichen sogenannten " waterless cleaner" hingewiesen, deren gute Reinigungswirkung vornehmlich auf den vorgenannten organischen Lösemitteln, insbesondere Benzine, Kerosine, kurzkettige Paraffinöle, beruhen. So weisen handelsübliche "waterless cleaners" die nachfolgend genannte Zusammensetzung auf:

| | |
|---|---|
| Petroleum Destillate: | 35,0 bis 45,0 Gew.-% |
| Wasser: | 30,0 bis 35,0 Gew.-% |
| Mineralöle: | 10,0 bis 20,0 Gew.-% |
| Natriumoleat: | 10,0 bis 20,0 Gew.-% |
| Trideceth-9: | 1,0 bis 5,0 Gew.-% |
| Propylengykol: | 1,0 bis 5,0 Gew.-% |
| Petrolatum: | 1,0 bis 5,0 Gew.-% |
| Lanolin: | 1,0 bis 5,0 Gew.-% |
| Zinkpyrithion: | 0,1 bis 1,0 Gew.-% |

Weitere Beispiele solcher lösungsmittelhaltigen "waterless cleaners" finden sich bei Ernest W. Flick, "Cosmetic and Toiletry Formulations", Second Edition, 1989, Seite 737 bis 744. Solche Grobhandreiniger werden ohne den Zusatz von Wasser eingesetzt, wobei die Reinigung ausschließlich mit dem Produkt und einem Trocknungstuch erfolgt.

Zur Entfernung von durch Druckfarben und/oder Tinten, insbesondere Druckertinten hervorgerufenen Haut- und Handreinigungsverschmutzungen sind Haut- und Handreinigungspräparate im Markt erhältlich, die Natriumdithionit und Cocamide DEA (Cocamide Diethanolamin) enthalten. Insbesondere das Produkt, daß unter dem Handelsnamen STOKOMIN II Stockhausen erhältlich ist, hat sich bei den durch Tinten hervorgerufenen Haut- und Handreinigungsverschmutzungen als sehr effektiv erwiesen.

Es wurde jedoch gefunden, daß der Grad der Schmutzentfernung in einem direkten Zusammenhang mit dem freien Gehalt an Diethanolamin steht, der herstellungsbedingt aus dem jeweilig verwendeten Cocamide DEA-Typ stammt. So führte beispielsweise der Einsatz von Cocamide DEA-Typen (Comperlan COD) mit einem freien Diethanolamin-Gehalt von < 2 Gew.-% im Endprodukt zu deutlich schlechteren Reinigungsergebnissen. Es wurde weiter gefunden, daß ein Gehalt an freiem Diethanolamin > 1,2 Gew.-% im Endprodukt bei Verwendung von Cocamide DEA-Typen notwendig ist, um eine effektive Beseitigung der durch Druckfarben und/oder Tinten; insbesondere Druckertinten hervorgerufenen Haut- und Handreinigungsverschmutzungen zu bewirken.

Aus wissenschaftlichen Studien ist jedoch bekannt, daß Diethanolamin ein gewisses Sensibilisierungsrisiko besitzt, weshalb die nationalen Gesetzgeber Grenzwerte festgesetzt haben, die den Einsatz von Diethanolamin in kosmetischen Produkten begrenzen. So bestimmt beispielsweise die deutsche Kosmetikverordnung, daß ein Gehalt an freiem Diethanolamin in Kosmetika von maximal 0,5 Gew.-%, bezogen auf das Endprodukt, nicht überschritten werden darf.

Im Hinblick auf die vielfältige Beschaffenheit der im Markt erhältlichen Druckfarben und/oder Tinten, insbesondere Druckertinten und die hierdurch verursachten hartnäckigen Haut- und Handreinigungsverschmutzungen, die der Reinigung mit konventionellen Hautreinigungsmitteln meist nicht zugänglich sind, besteht weiterhin ein Bedarf an Haut- bzw. Handreinigungsmitteln, die weitestgehend frei an freiem Diethanolamin im Reinigungsprodukt sind und die eine vergleichbare bzw. verbesserte Reinigungswirkung zeigen, wie die im Stand der Technik bekannten Produkte mit einem Gehalt an freiem Diethanolamin > 1,2 Gew.-% zur effektiven Beseitigung der durch Druckfarben und/oder Tinten, insbesondere Druckertinten hervorgerufenen Haut- und Handreinigungsverschmutzungen.

Aufgabe war es daher, Haut- bzw. auch Handreinigungsmittel, insbesondere zur Reinigung von extremen Haut- und Handreinigungsverschmutzungen, die beispielsweise durch reduzierbare oder oxidierbare Druckfarben und/oder Tinten hervorgerufen werden, bereitzustellen, die eine vergleichbare Reinigungswirkung aufweisen, wie die im Stand der Technik erhältlichen Präparate, jedoch einen Gehalt an freiem Diethanolamin von < 0,5 Gew.-% haben sollen. Darüberhinaus soll das Haut- und Handreinigungsmittel dergestalt stabilisiert sein, daß ein homogenes und stabiles Endprodukt entsteht.

Die Aufgabe wurde überraschend gelöst durch ein Haut- und Handreinigungsmittel, insbesondere zur Entfernung von Druckfarben und/oder Tinten, insbesondere Druckertinten, das die Komponenten
a.) mindestens 1 Gew.-%, mindestens ein ethoxyliertes Amin und/oder ethoxyliertes Diamin,
b.) 30 bis 70 Gew.-% mindestens ein Polyethylenglykol der allgemeinen, Formel H-O-(CH₂CH₂-O)ₙH,
   wobei n eine ganze Zahl von 1 bis 150 ist,
c.) 1 bis 30 Gew.-% mindestens einen Fettalkoholpolyglykolether,
d.) 0,1 bis 5 Gew.-% mindestens ein Komplexierungsmittel,
e.) 0 bis 30 Gew.-% mindestens ein Reduktions- oder Oxidationsmittel,
f.) 0 bis 25 Gew.-% eines oder mehrerer Abrasiva,
g.) 0 bis 10 Gew.-% mindestens einen mehrwertigen Alkohol,
h.) 0 bis 3 Gew.-% Wasser,
i.) gegebenenfalls eines oder mehrere viskositätsbildende Mittel,
j.) gegebenenfalls weitere kosmetische Hilfs-, Zusatz- und/oder Wirkstoffe,
wobei die Summe der Komponenten a.) bis j.) 100 Gew.-%, bezogen auf die Zusammensetzung des Reinigungsmittels, ergibt, aufweist.

Als Komponente a.) können hierbei insbesondere ethoxylierte Amine gemäß der allgemeinen Formel 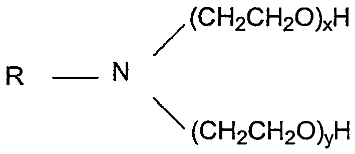 verwendet werden, wobei
R ein gesättigter, ungesättigter, verzweigter oder unverzweigter Alkylrest mit 1 bis 24 C-Atomen bedeutet und
x und y eine ganze Zahl von 1 bis 30 ist und x = y oder x ≠ y sein kann und die Summe von x+y ≤ 60 ist.

Vorzugsweise ist das ethoxylierte Amin aus der Gruppe der Oleylamine, Talgamine und Kokosamine ausgewählt, wobei besonders Oleylamine, Talgamine und Kokosamine besonders bevorzugt sind, die die Indizes x+y = 2, 5 oder 15, d.h. 2, 5 oder 15 EO-Einheiten aufweisen. Solche ethoxylierten tertiären aliphatischen Amine sind unter dem Handelsnamen Ethomeen® von der Firma AKZO NOBEL erhältlich. Exemplarisch sind hierzu folgende Handelsprodukte zu nennen:

| | |
|---|---|
| Ethomeen® C/12 | (INCI: PEG-2 Cocamin) |
| Ethomeen® C/15 | (INCI: PEG-5 Cocamin) |
| Ethomeen® C/25 | (INCI: PEG-15 Cocamin) |
| Ethomeen® 18/12 | (INCI: PEG-2 Stearamin) |
| Ethomeen® 18/15 | (INCI: PEG-5 Stearamin) |
| Ethomeen® 18/25 | (INCI: PEG-15 Stearamin) |
| Ethomeen® OV/12 | (INCI: PEG-2 Oleamin) |
| Ethomeen® S/12 | (INCI: PEG-2 Soyamin) |
| Ethomeen® S/15 | (INCI: PEG-5 Soyamin) |
| Ethomeen® S/25 | (INCI: PEG-15 Soyamin) |
| Ethomeen® T/12 | (INCI: PEG-2 Talgamin) |
| Ethomeen® T/25 | (INCI: PEG-15 Talgamin) |

Vorzugsweise werden Ethomeen® C/12, Ethomeen® S/12 und Ethomeen® T/12 als ethoxyliertes tertiäres Amin eingesetzt, wobei das Handelsprodukt Ethomeen® OV/12 erfindungsgemäß ganz besonders bevorzugt ist.

Ethoxylierte Diamine der Komponente a.) sind vorzugsweise Diamine gemäß der allgemeinen Formel II 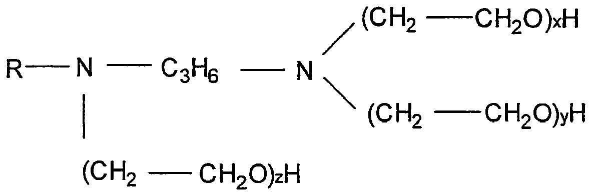 wobei
R = gesättigter, ungesättigter, verzweigter oder unverzweigter Alkylrest mit 1 bis 24 C-Atomen bedeutet und
x, y und z eine ganze Zahl von 1 bis 10 ist und x = y = z oder x, y und z verschieden voneinander sein kann und die Summe von x+y+z ≤ 30 ist.
Exemplarisch können hier die Handelsprodukte der Fa. Akzo Nobel, die unter dem Handelsnamen Ethoduomeen® erhältlich sind, genannt werden.

Erfindungsgemäß sind als Komponente b.) 30 bis 70 Gew.-%, vorzugsweise 40 bis 65 Gew.-%, besonders bevorzugt 50 bis 60 Gew.-%, bezogen auf die Zusammensetzung des Haut- und Handreinigungsmittels, mindestens ein Polyethylenglykol der allgemeinen Formel H-O-(CH₂CH₂-O)ₙH einsetzbar, wobei n eine ganze Zahl von 1 bis 150, vorzugsweise n eine ganze Zahl von 1 bis 25 ist

Vorteilhaft können Polyethylenglykole mit einem Molekulargewicht von 200 bis 1000 verwendet werden. Hierbei haben sich Polyethylenglykole mit einem Molekulargewicht von 400 als besonders vorteilhaft erwiesen. Solche Polyethylenglykole, die u.a. als technische Lösungsmittel mannigfaltig Anwendung finden, können beispielsweise von der Fa. BASF AG, Ludwigshafen, Deutschland unter dem Handelsnamen Lutrol® bezogen werden.

Weiterhin weist das erfindungsgemäß Haut- und Handreinigungsmittel als Komponenten c.) 1 bis 30 Gew.-%, vorzugsweise 1 bis 20 Gew.-% und besonders bevorzugt 3 bis 15 Gew.-%, bezogen auf die Zusammensetzung des Haut- und Handreinigungsmittels, mindestens einen Fettalkoholpolyglykolether auf. Solche Fettalkoholpolyglykolether, die u.a. als Emulgatoren, Netzmittel und Dispergiermittel in der chemisch-technischen Industrie Verwendung finden, sind nichtionische Tenside, die in bekannterweise durch Umsetzung von Ethylenoxid und beispielsweise Fettalkoholen erhältlich sind. Als Ausgangsmaterialien für die Herstellung dieser Fettalkoholpolyglykolether werden sowohl technisch hergestellte als auch native Fettalkohole verwendet. Hier sind beispielsweise Kokosfettalkohol und Oleylalkohol bzw. native Fettalkohole mit einer Jodzahl von 50, die 12 bis 18 oder überwiegend 18 Kohlenstoffatome im Alkoholmolekül aufweisen sind, als geeignete Fettalkohole zu nennen.

Die als Komponente c.) eingesetzten Fettalkoholethoxylate weisen vorzugsweise die allgemeine Formel

R-O-(CH₂CH₂-O)ₙH

auf, wobei
R ein gesättigter, ungesättigter, verzweigter oder unverzweigter Alkylrest und
n eine ganze Zahl von 1 bis 11 sind.

Vorzugsweise werden als gesättigter, ungesättigter, verzweigter oder unverzweigter Alkylrest ein Alkylrest mit C₈ bis C₁₈ Kohlenstoffatomen, insbesondere C₁₀ bis C₁₆ und besonders bevorzugt C₁₁ bis C₁₄ verwendet, wobei bevorzugt n eine ganze Zahl von 3 bis 10, insbesondere n eine ganze Zahl von 5 bis 7 ist.

Vorzugsweise können die erfindungsgemäßen Haut- und Handreinigungsmittel Laureth-6 als Fettalkoholethoxylat aufweisen.

In einer erfindungsgemäß besonders bevorzugten Ausführungsform können die Haut-und Handreinigungsmittel die unter der Marke INTRASOL® von der Fa. Sasol Servo Delden vertriebenen Fettalkoholpolyglykolether, vorzugsweise die nichtionischen Tenside mit der Bezeichnung INTRASOL® FA 12/18/5 enthalten, die auf einem Fettalkohol mit 12 bis 18 Kohlenstoffatomen basieren und 5 EO-Einheiten aufweisen.

Als Komponente d.) weisen die erfindungsgemäßen Haut- und Handreinigungsmittel zwingend 0,1 bis 5 Gew.-%, vorzugsweise 0,5 bis 4 Gew.-% bezogen auf die Zusammensetzung des Haut- und Handreinigungsmittels, mindestens einen Komplexbildner auf.
Komplexbildner bzw. Chelatbildner werden üblicherweise in der Kosmetik und der medizinischen Galenik als Hilfsstoffe verwendet und dienen dazu, durch Komplexierung störender Metallionen unerwünschte chemische Reaktionen in kosmetischen oder pharmazeutischen Zubereitungen zu verhindern.

In den erfindungsgemäßen Haut- und Handreinigungsmittel haben die Komplex- oder auch Chelatbildner u.a. die Aufgabe, die für die Haut- und Handverschmutzungen verantwortlichen Druckfarben und/oder Tintenfarbstoffe in lösliche Komplexe zu überführen, so daß sie auf der Haut wirksam entfernt werden können. Erfindungsgemäß sind daher alle Komplexbildner einsetzbar, die zur Komplexierung bzw. auch Maskierung von Druckfarb- und/oder Tintenfarbstoffen einsetzbar sind. Bekannte Komplexbildner sind beispielsweise Polycarbonsäuren, Polyamine, Kronenether, Kryptanden etc. Insbesondere können Wein- und Citronensäure und deren Salze, Aminopolycarbonsäuren und deren Salze, wie z.B. Ethylendiamintetraessigsäure (EDTA), Nitriloessigsäure (NTA), Hydroxyethylendiaminotriessigsäure (HOEDTA) und deren Salze, Diethylenaminopentaessigsäure und deren Salze (DPTA), Methylglycindiessigsäure (MGDA) und deren Salze, Iminodibernsteinsäure deren Salze, trans-1,2-Diaminocyclohexantetraessigsäure (CDTA) und deren Salze, Polyasparaginsäure und deren Salze aber auch Gerüststoffe, sogenannte Builder und Cobuilder wie z.B. Polycarboxylate oder Polyphosphate verwendet werden. Vorzugsweise weisen die erfindungsgemäß Haut- und Handreinigungsmittel EDTA, das Tetra-Natriumsalz der Iminodibernsteinsäure (Tetra-Natrium Iminodisuccinat) und Natiumpolyaspartat als Komplexbildner auf, wobei ein Gehalt des Komplexbildners bzw. Komplexbildnergemisches von 2 Gew.-%, bezogen auf das Haut- und Handreinigungsmittel, besonders bevorzugt ist.

Neben den Komponenten a.) bis d.), die die erfindungsgemäßen Haut- und Handreinigungsmittel zwingend aufweisen, können die Haut- und Handreinigungsmittel optional weitere Komponenten e.) bis j.) aufweisen, die das Reinigungsergebnis vorteilhaft verbessern können.

So können die erfindungsgemäß Haut- und Handreinigungsmittel abhängig von der Art der zu entfernenden Haut- bzw. Handverschmutzung als Komponente e.) 0 bis 30, vorzugsweise 1 bis 25 Gew.-% und besonders bevorzugt 3 bis 20 Gew.-% mindestens ein Reduktions- oder Oxidationsmittel aufweisen. Zur wirksamen Entfernung einer Vielzahl von reduzierbaren Druckfarben und/oder Tinten, insbesondere Druckertinten können Dithionite bzw. Hydrosulfite wie z.B. Natriumdithionit verwendet werden, die schon seit Jahren für diesen Einsatzzweck bekannt sind. In einer besonders bevorzugten Ausführungsform der Erfindung weisen die erfindungsgemäß Haut- und Handreinigungsmittel 8 bis 12 Gew.-% Natriumdithionit als Reduktionsmittel auf.

Als Oxidationsmittel können peroxidfreisetzende Verbindungen eingesetzt werden. Solche Oxidationsmittel werden beispielsweise in der US 2002/0013237 A1 genannt . Die US 2002/0013237 A1 betrifft Hautreinigungsmittel, um Tinten und andere Flecken von Armen und Händen zu entfernen, die wirksame Mengen eines niedermolekularen einwertigen Alkohols mit 1 bis 12 Kohlenstoffatomen und an einem peroxidfreisetzenden Agens, wie z.B. ein Perboratsalz, vorzugsweise Natriumperborat aufweisen, so daß durch eine synergistische Reaktion des Alkohols mit dem Perboratsalz eine Entfernung der Tinte von der Haut bewirkt werden soll. Insbesondere soll lediglich bei Kontakt des Reinigungsmittels mit der Tintenverschmutzung diese synergistische Reaktion ausgelöst werden, die dann für die Entfernung der Tintenverschmutzung verantwortlich ist. Die dort beschriebenen Haut- und Handreinigungsmittel enthalten 40 bis 80 Gew.-%, bezogen auf die Gesamtmenge des Reinigungsmittels, vorzugsweise Ethanol oder Isopropanol, insbesondere weil mehrwertige Alkohole, aber auch ether- und estersubstituierte Alkohole nicht in dem Maße eine synergistische Reaktion bewirken, um eine wirksame Entfernung der Tintenverschmutzungen zu gewährleisten.
Es ist in diesem Zusammenhang zu betonen, daß die erfindungsgemäßen Haut- und Handreinigungsmittel, die die Komponenten a.) bis d.) aufweisen, eine effektive Entfernung der durch Druckfarben und Tinten verursachten Haut- und Handverschmutzungen bewirken, wobei die Reinigungswirkung bzw. die Farbstoffentfernung vornehmlich auf das Vorhandensein der Komponente a.), d.h. dem Anteil an ethoxyliertem Amin und/oder ethoxyliertem Diamin zurückzuführen sein dürfte bzw. es ist von einem synergistischen Zusammenwirken der Komponenten a.) bis d.) auszugehen. Darüberhinaus weisen die erfindungsgemäßen Haut- und Handreinigungsmittel keine einwertigen Alkohole wie z.B. Ethanol oder Isopropanol mit einem Gehalt ≥ 40 Gew.-%, bezogen auf die Gesamtmenge des Reinigungsmittels, auf, so daß eine synergistische Reaktion entsprechend der US 2002/0013237 A1 nicht stattfinden kann, wenn ein erfindungsgemäßes Haut- und Handreinigungsmittel als unterstützende Reinigungskomponente als Oxidationsmittel ein Perboratsalz, wie z.B. Natriumperborat aufweist.

Obgleich die erfindungsgemäßen Haut- und Handreinigungsmittel eine sehr gute Reinigungswirkung zeigen, so daß der Zusatz von Abrasiva zu diesen Reinigungsmitteln entbehrlich ist, können die Haut- und Handreinigungsmittel gegebenenfalls für bestimmte Reinigungsanwendungen vorteilhaft Abrasiva als Komponente f.) enthalten. Der Anteil des Abrasivums oder der Abrasiva kann dann 0 bis 25 Gew.-%, bezogen auf die Zusammensetzung des Reinigungsmittels, bevorzugt 10 bis 20 Gew.-% betragen.

Bevorzugt zu verwendende Abrasiva sind beispielsweise Kunststoffreibemittel auf der Basis von Polyethylen oder Polyurethan, Pflanzenmehle wie z.B. Maiskolbenmehl, Weizenkleie, Hafermehl und Holzmehl, Reibemittel auf der Basis von natürlichen Kern-und/oder Schalenmehlen, insbesondere Walnußschalen-, Mandelschalen-, Haselnußschalen-, Olivenkern-, Aprikosenkern- und Kirschkernmehl oder beliebige Gemische dieser Schalen- und Kemmehle und Perlen aus Wachsen, wie z.B. Jojobawachse, wobei gebleichte Mehle, insbesondere mit Wasserstoffperoxid gebleichtes Walnußschalenmehl besonders bevorzugt ist, das vorteilhaft zur Entfernung von Pigmentverschmutzungen der jeweiligen Druckfarben bzw. Druckertinten verwendet werden kann.

Als Komponente g.) weisen die erfindungsgemäßen Haut- und Handreinigungsmittel mindestens 0 bis 10 Gew.-%, vorzugsweise 1 bis 5 Gew.-% einen mehrwertigen Alkohol auf. Solche mehrwertige Alkohole sind beispielsweise geradkettige, verzweigte oder cyclische Alkanole mit 2 bis 12, vorzugsweise 2 bis 6 Kohlenstoffatomen, wobei Glyzerin und/oder 1,2 Propandiol besonders bevorzugt sind.

In einer weiteren Ausführungsform der Erfindung können die erfindungsgemäßen Haut-und Handreinigungsmittel gegebenenfalls 0 bis 3 Gew.-% Wasser als Komponente h.) bzw. 0 bis 10 Gew.-% eines oder mehrere viskositätsbildende Mittel als Komponente i.) aufweisen. Solche viskositätsbildenden Mittel bzw. Verdickungsmittel sind beispielsweise organophile und/oder hydrophile Schichtsilikate, insbesondere Bentonite, Polysaccharide, wie z.B. Cellulose, Guarmehl und/oder Xanthane, modifizierte Polysaccharide, bevorzugt Celluloseether, Carboxymethylcellulose und/oder Hydroxyalkylcellulosen, vorzugsweise Hydroxyethylcellulose, Alginate und/oder anorganische Elektrolyte, vorzugsweise Natriumchlorid und/oder Magnesiumsulfat und/oder pyrogene Kieselsäuren, die beispielsweise von der Firma Degussa AG unter dem Handelsnamen Aerosil® erhältlich sind. Vorzugsweise können die Haut- und Handreinigungsmittel 0 bis 5 Gew.-%, vorzugsweise 0 bis 3 Gew.-% modifizierte hydrophobe Cellulosen wie z.B. Cetyl Myristyl Hydroxyethyl Ethylcellulose aufweisen, die unter dem Handelsnamen Elfacos® CDHM von der Firma AKZO NOBEL bezogen werden könnnen. Besonders bevorzugt ist eine Kombination von pyrogener Kieselsäure, wie z.B. Aerosil® 200 und den modifizierten hydrophoben Cellulosen vom Elfacos®-Typ.

Die erfindungsgemäßen Haut- und Handreinigungsmittel können als Komponenten j.) gegebenenfalls weitere kosmetische Hilfs-, Zusatz- und/oder Wirkstoffe, beispielsweise pH-Regulatoren, Stabilisatoren, vorzugsweise Cetearylalkohol und/oder hydrierte Ricinusöle, wie z.B. Trihydroxystearin, Duftstoffe, Konservierungsmittel, bevorzugt organische Säuren und Antioxidantien, wie z.B. Vitamin E-Acetat und/oder ölige oder wässrige Pflegekomponenten enthalten.

Die Herstellung der erfindungsgemäßen Haut- und Handreinigungsmittel, insbesondere Grobhandreiniger erfolgt üblicherweise mittels bekannter Vorrichtungen im Batch- oder kontinuierlichen Verfahren, wobei die Haut- und Handreinigungsmittel vorzugsweise als cremige Mittel oder als fließfähige viskose Pasten erhalten werden. Geeignete Vorrichtungen sind temperierbare Kessel mit Rührwerk, Mischer und z.B. auch Extruder.

Erfindungsgemäß bevorzugte Haut- und Handreinigungsmittel haben folgende Zusammensetzung:

| Komponente | | Gew.-% |
|---|---|---|
| a.) | Ethoduomeen OV 13 und/oder Ethomeen OV 12 | 10,0 bis 20,0 Gew.-% |
| b.) | Polydiol 400 | 40,0 bis 60,0 Gew.-% |
| c.) | Intrasol FA 1218/10 | 5,0 bis 10,0 Gew.-% |
| d.) | EDTA | 1,0 bis 2,0 Gew.-% |
| e.) | Na-Dithionit | 5,0 bis 15,0 Gew.-% |
| f.) | Wainußschafenmehl, gebleicht | 5,0 bis 10 Gew.-% |
| g^{.}) | Propandiol1,2 und/oder Glyzerin | 1,0 bis 3 Gew.-% |
| i.) | Elfacos® CDHM | 0,5 bis 1,0 Gew.-% |
| | Aerosil® 200 | 1,0 bis 3,0 Gew.-% |
| j.) | Parfümöl | 0,5 Gew.-% |

Es konnte überraschend gezeigt werden, daß durch Verwendung solcher Haut- und Handreinigungsmittel, die einen Gehalt an freiem Diethanolamin von < 0,5 Gew.-% aufweisen, diese eine vergleichbare Reinigungswirkung erzielen als beispielsweise handelsübliche Produkte, die einen Gehalt an freiem Diethanolamin von bis zu 2,8 Gew.-% aufweisen.

## Patentansprüche

1. Hautreinigungsmittel, insbesondere zur Entfernung von Druckfarben und/oder Tinten, **dadurch gekennzeichnet, daß** es die Komponenten
a.) mindestens 1 Gew.-%, mindestens ein ethoxyliertes Amin und/oder ethoxyliertes Diamin,
b.) 30 bis 70 Gew.-% mindestens ein Polyethylenglykol der allgemeinen Formel H-O-(CH₂CH₂-O)ₙH,
wobei n eine ganze Zahl von 1 bis 150 ist,
c.) 1 bis 30 Gew.-% mindestens einen Fettalkoholpolyglykolether,
d.) 0,1 bis 5 Gew.-% mindestens ein Komplexierungsmittel,
e.) 0 bis 30 Gew.-% mindestens ein Reduktions- oder Oxidationsmittel,
f.) 0 bis 25 Gew.-% eines oder mehrerer Abrasiva,
g.) 0 bis 10 Gew.-% mindestens einen mehrwertigen Alkohol,
h.) 0 bis 3 Gew.-% Wasser,
i.) gegebenenfalls eines oder mehrere viskositätsbildende Mittel,
j.) gegebenenfalls weitere kosmetische Hilfs-, Zusatz- und/oder Wirkstoffe,
wobei die Summe der Komponenten a.) bis j.) 100 Gew.-%, bezogen auf die Zusammensetzung des Reinigungsmittels, ergibt, aufweist.

2. Hautreinigungsmittel nach Anspruch 1, **dadurch gekennzeichnet, daß** das ethoxylierte Amin ein Amin gemäß der allgemeinen Formel I 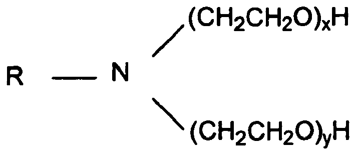 ist, wobei
R = gesättigter, ungesättigter, verzweigter oder unverzweigter Alkylrest mit 1 bis 24 C-Atomen bedeutet und
x und y eine ganze Zahl von 1 bis 30 ist und x = y oder x ≠ y sein kann, wobei die Summe von x+y ≤ 60 ist.

3. Hautreinigungsmittel nach Anspruch 1 oder 2, **dadurch gekennzeichnet, daß** das ethoxyliertes Amin aus der Gruppe der Oleylamine, Talgamine und Kokosamine ausgewählt ist.

4. Hautreinigungsmittel nach einem der Ansprüche 1 bis 3, **dadurch gekennzeichnet, daß** das ethoxylierte Diamin ein Diamin gemäß der allgemeinen Formel II 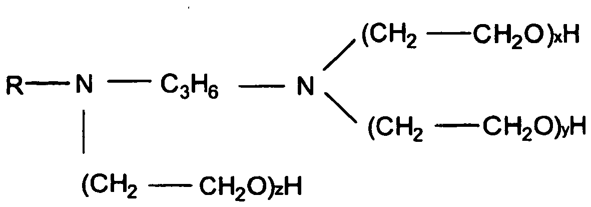 ist, wobei
R = gesättigter, ungesättigter, verzweigter oder unverzweigter Alkylrest mit 1 bis 24 C-Atomen bedeutet und
x, y und z eine ganze Zahl von 1 bis 10 ist und x = y = z oder x, y und z verschieden voneinander sein kann, wobei die Summe von x+y+z ≤ 30 ist.

5. Hautreinigungsmittel nach einem der Ansprüche 1 bis 4, **dadurch gekennzeichnet, daß** es als Komponente b.) 40 bis 65 Gew.-% mindestens ein Polyethylenglykol der allgemeinen Formel H-O-(CH₂CH₂-O)ₙH, wobei n eine ganze Zahl von 1 bis 150 ist, aufweist.

6. Hautreinigungsmittel nach Anspruch 5, **dadurch gekennzeichnet, daß** es als Komponente b.) 50 bis 60 Gew.-% mindestens ein Polyethylenglykol der allgemeinen Formel H-O-(CH₂CH₂-O)ₙH, wobei n eine ganze Zahl von 1 bis 25 ist, aufweist.

7. Hautreinigungsmittel nach einem der Ansprüche 1 bis 6, **dadurch gekennzeichnet, daß** es als Komponente c.) 1 bis 20 Gew.-% mindestens einen Fettalkoholpolyglykolether aufweist.

8. Hautreinigungsmittel nach Anspruch 7, **dadurch gekennzeichnet, daß** die Komponente c.) wenigstens ein Fettalkoholethoxylat der allgemeinen Formel
R-O-(CH₂CH₂O)ₙH
ist, wobei
R ein gesättigter, ungesättigter, verzweigter oder unverzweigter Alkylrest,
n eine ganze Zahl von 1 bis 11 sind.

9. Hautreinigungsmittel nach einem der Ansprüche 1 bis 8, **dadurch gekennzeichnet, daß** es als Komponente d.) 0,1 bis 4 Gew.-% mindestens einen Komplexbildner aufweist.

10. Hautreinigungsmittel nach einem der Ansprüche 1 bis 9, **dadurch gekennzeichnet, daß** es als Komponente d.) 2 Gew.-% mindestens einen Komplexbildner aufweist.

11. Hautreinigungsmittel nach einem der Ansprüche 1 bis 10, **dadurch gekennzeichnet, daß** es als Komponente e.) 1 bis 25 Gew.-% mindestens ein Reduktions- oder Oxidationsmittel aufweist.

12. Hautreinigungsmittel nach Anspruch 11, **dadurch gekennzeichnet, daß** es als Reduktionsmittel Dithionite aufweist.

13. Hautreinigungsmittel nach Anspruch 12, **dadurch gekennzeichnet, daß** es als Reduktionsmittel 8 bis 12 Gew.-% Natriumdithionit aufweist.

14. Hautreinigungsmittel nach Anspruch 11, **dadurch gekennzeichnet, daß** es als Oxidationsmittel wenigstens ein Perboratsalz aufweist.

15. Hautreinigungsmittel nach einem der Ansprüche 1 bis 14, **dadurch gekennzeichnet, daß** es als Komponente f.) 10 bis 20 Gew.-% an Abrasiva aufweist.

16. Hautreinigungsmittel nach einem der Ansprüche 1 bis 15, **dadurch gekennzeichnet, daß** es als Komponente g.) 1 bis 5 Gew.-% mindestens einen mehrwertigen Alkohol aufweist, der ein geradkettiges, verzweigtes oder cyclisches Alkanol mit 2 bis 12 Kohlenstoffatomen ist.

17. Hautreinigungsmittel nach einem der Ansprüche 1 bis 16, **dadurch gekennzeichnet, daß** es als Komponente i.) als viskositätsbildendes Mittel eine Kombination von Cetyl Myrityl Hydroxyalkylcellulose und pyrogene Kieselsäure aufweist.

18. Hautreinigungsmittel nach einem der Ansprüche 1 bis 17, **dadurch gekennzeichnet, daß** es als Komponente j.) als kosmetische Hilfs-, Zusatz- und/oder Wirkstoffe pH-Regulatoren, Stabilisatoren, Duftstoffe, Konservierungsmittel, Antioxidantien und/oder ölige oder wässrige Pflegekomponenten aufweist

## Claims

1. Skin cleansing agent, particularly for removing printing inks and/or inks, **characterized in that** it has the components
a.) at least 1% by weight, at least one ethoxylated amine and/or ethoxylated diamine,
b.) 30 to 70% by weight of at least one polyethylene glycol of the general formula H-O- (CH₂CH₂-O)ₙH,
where n is an integer from 1 to 150,
c.) 1 to 30% by weight of at least one fatty alcohol polyglycol ether,
d.) 0.1 to 5% by weight of at least one complexing agent,
e.) 0 to 30% by weight of at least one reducing agent or oxidizing agent,
f.) 0 to 25% by weight of one or more abrasives,
g.) 0 to 10% by weight of at least one polyhydric alcohol,
h.) 0 to 3% by weight of water,
i.) optionally one or more viscosity-forming agents,
j.) optionally further cosmetic auxiliaries, additives and/or active ingredients,
where the sum of components a.) to j.) gives 100% by weight, based on the composition of the cleansing agent.

2. Skin cleansing agent according to Claim 1, **characterized in that** the ethoxylated amine is an amine according to the general formula I 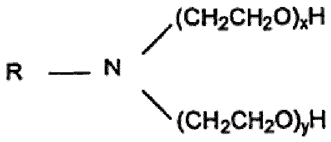 where
R = saturated, unsaturated, branched or unbranched alkyl radical having 1 to 24 carbon atoms and
x and y is an integer from 1 to 30 and x = y or x ≠ y, where the sum of x + y is ≤ 60.

3. Skin cleansing agent according to Claim 1 or 2, **characterized in that** the ethoxylated amine is selected from the group of oleylamines, tallow amines and coconut amines.

4. Skin cleansing agent according to one of Claims 1 to 3, **characterized in that** the ethoxylated diamine is a diamine according to the general formula II 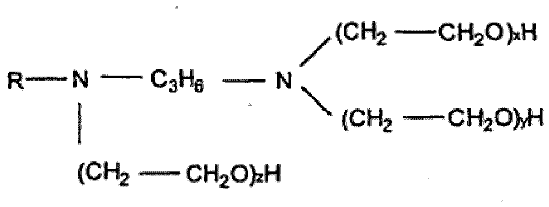 where
R = saturated, unsaturated, branched or unbranched alkyl radical having 1 to 24 carbon atoms and
x, y and z is an integer from 1 to 10 and x = y = z or x, y and z may be different from one another where the sum of x + y + z is ≤ 30.

5. Skin cleansing agent according to one of Claims 1 to 4, **characterized in that** it has, as component b.), 40 to 65% by weight of at least one polyethylene glycol of the general formula H-O-(CH₂CH₂-O)ₙH, where n is an integer from 1 to 150.

6. Skin cleansing agent according to Claim 5, **characterized in that** it has, as component b.), 50 to 60% by weight of at least one polyethylene glycol of the general formula H-O-(CH₂CH₂-O)ₙH, where n is an integer from 1 to 25.

7. Skin cleansing agent according to one of Claims 1 to 6, **characterized in that** it has, as component c.), 1 to 20% by weight of at least one fatty alcohol polyglycol ether.

8. Skin cleansing agent according to Claim 7, **characterized in that** component c.) is at least one fatty alcohol ethoxylate of the general formula
R-O-(CH₂CH₂O)ₙH
where
R is a saturated, unsaturated, branched or unbranched alkyl radical,
n is an integer from 1 to 11.

9. Skin cleansing agent according to one of Claims 1 to 8, **characterized in that** it has, as component d.), 0.1 to 4% by weight of at least one complex former.

10. Skin cleansing agent according to one of Claims 1 to 9, **characterized in that** it has, as component d.), 2% by weight of at least one complex former.

11. Skin cleansing agent according to one of Claims 1 to 10, **characterized in that** it has, as component e.), 1 to 25% by weight of at least one reducing agent or oxidizing agent.

12. Skin cleansing agent according to Claim 11, **characterized in that** it has dithionites as reducing agent.

13. Skin cleansing agent according to Claim 12, **characterized in that** it has 8 to 12% by weight of sodium dithionite as reducing agent.

14. Skin cleansing agent according to Claim 11, **characterized in that** it has at least one perborate salt as oxidizing agent.

15. Skin cleansing agent according to one of Claims 1 to 14, **characterized in that** it has 10 to 20% by weight of abrasives as component f.).

16. Skin cleansing agent according to one of Claims 1 to 15, **characterized in that** it has, as component g.), 1 to 5% by weight of at least one polyhydric alcohol, which is a straight-chain, branched or cyclic alkanol having 2 to 12 carbon atoms.

17. Skin cleansing agent according to one of Claims 1 to 16, **characterized in that** it has, as component i.), a combination of cetyl myristyl hydroxyalkylcellulose and fumed silica as viscosity-forming agent.

18. Skin cleansing agent according to one of Claims 1 to 17, **characterized in that** it has, as component j.), pH regulators, stabilizers, fragrances, preservatives, antioxidants and/or oily or aqueous care components as cosmetic auxiliaries, additives and/or active ingredients.

## Revendications

1. Produit de nettoyage pour la peau, en particulier pour l'élimination d'encres d'impression et/ou d'encres, **caractérisé en ce qu'**il comporte les composants
a.) au moins 1 % en poids d'au moins une amine éthoxylée et/ou diamine éthoxylée,
b.) 30 à 70 % en poids d'au moins un polyéthylèneglycol de formule générale H-O-(CH₂CH₂-O)ₙH,
n étant un nombre entier valant de 1 à 150,
c.) 1 à 30 % en poids d'au moins un polyglycoléther d'alcool gras,
d.) 0,1 à 5 % en poids d'au moins un complexant,
e.) 0 à 30 % en poids d'au moins un oxydant ou réducteur,
f.) 0 à 25 % en poids d'un ou plusieurs abrasifs,
g.) 0 à 10 % en poids d'au moins un alcool polyhydrique,
h.) 0 à 3 % en poids d'eau,
i.) éventuellement un ou plusieurs agents augmentant la viscosité,
j.) éventuellement d'autres adjuvants, additifs et/ou substances actives cosmétiques,
la somme des composants a.) à j.) étant égale à 100 % en poids, par rapport à la composition du produit de nettoyage.

2. Produit de nettoyage pour la peau selon la revendication 1, **caractérisé en ce que** l'amine éthoxylée est une amine de formule générale I 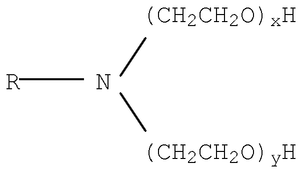 dans laquelle
R représente un radical alkyle saturé, insaturé, ramifié ou non ramifié, ayant de 1 à 24 atomes de carbone et
x et y représentent chacun un nombre entier valant de 1 à 30 et x = y ou x ≠ y, le somme de x+y étant ≤ 60.

3. Produit de nettoyage pour la peau selon la revendication 1 ou 2, **caractérisé en ce que** l'amine éthoxylée est choisie dans le groupe des oléylamines, amines de suif et amines de coco.

4. Produit de nettoyage pour la peau selon l'une quelconque des revendications 1 à 3, **caractérisé en ce que** l' amine éthoxylée est une diamine selon la formule générale II 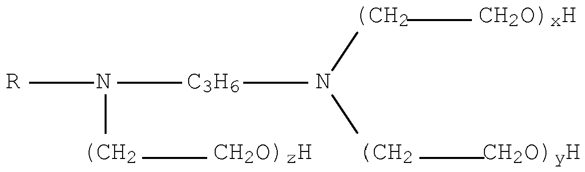 dans laquelle
R représente un radical alkyle saturé, insaturé, ramifié ou non ramifié, ayant de 1 à 24 atomes de carbone et
x, y et z représentent chacun un nombre entier valant de 1 à 10 et x = y = z ou x, y et z peuvent être différents entre eux, le somme de x+y+z étant ≤ 30.

5. Produit de nettoyage pour la peau selon l'une quelconque des revendications 1 à 4, **caractérisé en ce qu'**il comporte en tant que composant b.) 40 à 65 % en poids d'au moins un polyéthylèneglycol de formule générale H-O-(CH₂CH₂-O)ₙH, n étant un nombre entier valant de 1 à 150.

6. Produit de nettoyage pour la peau selon la revendication 5, **caractérisé en ce qu'**il comporte en tant que composant b.) 50 à 60 % en poids d'au moins un polyéthylèneglycol de formule générale H-O-(CH₂CH₂-O)ₙH, n étant un nombre entier valant de 1 à 25.

7. Produit de nettoyage pour la peau selon l'une quelconque des revendications 1 à 6, **caractérisé en ce qu'**il comporte en tant que composant c.) 1 à 20 % en poids d'au moins un polyglycoléther d'alcool gras.

8. Produit de nettoyage pour la peau selon la revendication 7, **caractérisé en ce que** le composant c.) est au moins un produit d'éthoxylation d'alcool gras, de formule générale
R-O-(CH₂CH₂O)ₙH
dans laquelle
R est un radical alkyle saturé, insaturé, ramifié ou non ramifié,
n est un nombre entier valant de 1 à 11.

9. Produit de nettoyage pour la peau selon l'une quelconque des revendications 1 à 8, **caractérisé en ce qu'**il comporte en tant que composant d.) 0,1 à 4 % en poids d'au moins un complexant.

10. Produit de nettoyage pour la peau selon l'une quelconque des revendications 1 à 9, **caractérisé en ce qu'**il comporte en tant que composant d.) 2 % en poids d'au moins un complexant.

11. Produit de nettoyage pour la peau selon l'une quelconque des revendications 1 à 10, **caractérisé en ce qu'**il comporte en tant que composant e.) 1 à 25 % en poids d'au moins un oxydant ou réducteur.

12. Produit de nettoyage pour la peau selon la revendication 11, **caractérisé en ce qu'**il comporte en tant que réducteur des dithionites.

13. Produit de nettoyage pour la peau selon la revendication 12, **caractérisé en ce qu'**il comporte en tant que réducteur 8 à 12 % en poids de dithionite de sodium.

14. Produit de nettoyage pour la peau selon la revendication 11, **caractérisé en ce qu'**il comporte en tant qu'oxydant au moins un sel de type perborate.

15. Produit de nettoyage pour la peau selon l'une quelconque des revendications 1 à 14, **caractérisé en ce qu'**il comporte en tant que composant f.) 10 à 20 % en poids d'abrasifs.

16. Produit de nettoyage pour la peau selon l'une quelconque des revendications 1 à 15, **caractérisé en ce qu'**il comporte en tant que composant g.) 1 à 5 % en poids d'au moins un alcool polyhydrique qui est un alcanol à chaîne droite, ramifié ou cyclique ayant de 2 à 12 atomes de carbone.

17. Produit de nettoyage pour la peau selon l'une quelconque des revendications 1 à 16, **caractérisé en ce qu'**il comporte comme agent augmentant la viscosité en tant que composant i.) une association de cétyl-myristyl-hydroxyalkylcellulose et d'acide silicique pyrogéné.

18. Produit de nettoyage pour la peau selon l'une quelconque des revendications 1 à 17, **caractérisé en ce qu'**il comporte comme adjuvants, additifs et/ou substances actives cosmétiques en tant qu'composant j.) des régulateurs de pH, des stabilisants, des parfums, des conservateurs, des antioxydants et/ou des composants de soin aqueux ou huileux.
